(19) Europäisches Patentamt
European Patent Office
Office européen des brevets

(11) EP 2 335 709 A1

(12) EUROPEAN PATENT APPLICATION

(43) Date of publication:
**22.06.2011 Bulletin 2011/25**

(21) Application number: **11002551.7**

(22) Date of filing: **25.03.2005**

(51) Int Cl.:
***A61K 31/785*** (2006.01)
***A61K 31/74*** (2006.01)
***A61K 31/787*** (2006.01)
***A61K 45/00*** (2006.01)
***A61P 3/06*** (2006.01)
***A61P 3/10*** (2006.01)
***A61P 5/50*** (2006.01)
***A61P 9/00*** (2006.01)
***A61P 9/10*** (2006.01)
***A61P 9/12*** (2006.01)
***A61P 13/12*** (2006.01)

(84) Designated Contracting States:
**AT BE BG CH CY CZ DE DK EE ES FI FR GB GR HU IE IS IT LI LT LU MC NL PL PT RO SE SI SK TR**

(30) Priority: **26.03.2004 JP 2004091508**

(62) Document number(s) of the earlier application(s) in accordance with Art. 76 EPC:
**05721449.6 / 1 733 732**

(71) Applicant: **Mitsubishi Tanabe Pharma Corporation**
**Osaka-shi**
**Osaka 541-8505 (JP)**

(72) Inventors:
- **Suzuki, Kazuo**
**Tokyo 103-8405 (JP)**
- **Nakajima, Shigekazu**
**Tokyo 103-8405 (JP)**
- **Sugimoto, Kanami**
**Tokyo 103-8405 (JP)**

(74) Representative: **TER MEER - STEINMEISTER & PARTNER GbR**
**Mauerkircherstraße 45**
**DE-81679 München (DE)**

Remarks:
This application was filed on 28-03-2011 as a divisional application to the application mentioned under INID code 62.

# (54) Insulin resistance improving agent

(57) The present invention relates to an agent for prophylactic, improving and/or therapeutic treatment of a disease or symptom resulting from insulin resistance, which comprises a pharmaceutically acceptable anion exchange resin as an active ingredient, wherein the disease or symptom resulting from insulin resistance is selected from the group consisting of abnormal lipid metabolism, arteriosclerosis, abnormal vascular endothelial function, coronary artery disease, cardiovascular disease, renal dysfunction, hypertension, type 2 diabetes, hyperuricemia, multiple risk factor syndrome, and gestational diabetes.



Printed by Jouve, 75001 PARIS (FR)

## Description

### Technical Field

The present invention relates to an insulin resistance-improving agent comprising a pharmaceutically acceptable anion exchange resin as an active ingredient.

### Background Art

As for anion exchange resins known as hypocholesterolemic agents, including colestimide (trade name: Cholebine, Mitsubishi Pharma Corporation) as a typical example, reports were made on hypoglycemic action after administration over a certain period of time (Non-patent document 1) and an effect on the circadian variation of blood glucose levels in hypercholesterolemia patients also suffering from type 2 diabetes (Patent document 1). However, modes of mechanisms thereof remain unknown. For example, cholestyramine resin is reported to have a hypoglycemic action (Non-patent document 2), and the mechanism is considered to be not associated with improvement of insulin resistance, but with inhibition of gastrointestinal motility via cholecystokinin (CCK) and promotion of insulin secretion.
Insulin preparations, sulfonylurea agents, and rapid-acting insulin secretagogues, which exhibit hypoglycemic actions, basically have no insulin resistance-improving action. $\alpha$-Glucosidase inhibitors that delay glucose absorption have only a slight indirect insulin resistance-improving action. Therefore, agents having a hypoglycemic action are not generally considered to have an insulin resistance-improving action. Since these agents are non-absorbable, it is believed that an artisan would not have been able to relate the agents to improvement of resistance in the peripheral muscles and the like or improvement in the liver.
At present, a class of glitazone antidiabetic drugs are known as typical examples of insulin resistance-improving agents. From a viewpoint of mode of action, the glitazone drugs have an adipocyte differentiation-inducing action, which action improves glucose uptake ability of adipocytes to lower the blood,glucose level. However, as an adverse reaction, the drugs increases adipose tissues (i.e., aggravation of obesity). In addition, since retention of humor (edema) was reported, these drugs are requested to be administered carefully to patients with decreased cardiac function frequently observed in diabetes patients. Further, since they have a risk of hepatotoxicity, liver function tests should be frequently performed.
Therefore, a novel medicament that solves the aforementioned problems, specifically, a non-absorbable medicament that improves insulin resistance without the increase of body weight (fat) and can be used without the risk of inducing edema or hepatotoxicity has been desired.
Further, to the best knowledge of the inventors of the present invention, no report has been made so far that pharmaceutically acceptable anion exchange resins exhibit an insulin resistance-improving action.

Patent document 1: WO03/011398
Non-patent document 1: Rinsho Iyaku (Journal of Clinical Therapeutics & Medicine), Vol. 12, No. 8, June 1996, p.1641
Non-patent document 2: Ann. Intern. Med., 1994; 121: pp.416-422

### Disclosure of the Invention

### Object to be Achieved by the Invention

Accordingly, an object of the present invention is to provide an insulin resistance-improving agent comprising a pharmaceutically acceptable anion exchange resin as an active ingredient.

### Means for Achieving the Object

The inventors of the present invention conducted various researches to achieve the foregoing object. As a result, they found that colestimide and colesevelam hydrochloride known as hypocholesterolemic agents and sevelamer hydrochloride known as a hyperphosphatemia curing agent exhibited an apparent insulin resistance-improving action and thus achieved the present invention.
The gists of the present invention are as follows:

1. An insulin resistance-improving agent comprising a pharmaceutically acceptable anion exchange resin as an active ingredient.
2. The insulin resistance-improving agent according to the aforementioned 1, wherein the pharmaceutically acceptable anion exchange resin has a bile acid-adsorbing ability.

3. The insulin resistance-improving agent according to the aforementioned 1 or 2, wherein the pharmaceutically acceptable anion exchange resin is selected from colestimide, cholestyramine resin, colestipol, sevelamer hydrochloride, and colesevelam hydrochloride.
4. The insulin resistance-improving agent according to the aforementioned 1 or 2, wherein the pharmaceutically acceptable anion exchange resin is an anion exchange resin synthesized by a polymerization reaction of an epichlorohydrin derivative and an amine of which typical example includes an imidazole derivative.
5. The insulin resistance-improving agent according to any one of the aforementioned 1 to 4, wherein the pharmaceutically acceptable anion exchange resin is colestimide.
6. The insulin resistance-improving agent according to any one of the aforementioned 1 to 5, with which an oral hypoglycemic agent is used simultaneously, separately, or successively.
7. The insulin resistance-improving agent according to the aforementioned 6, wherein the oral hypoglycemic agent is selected from the group consisting of $\alpha$-glucosidase inhibitors, biguanides, insulin sensitivity improving agents, sulfonylurea agents, rapid-acting insulin secretagogues, pharmaceutical preparations comprising GLP-1 or derivatives thereof, and DPP-IV inhibitors.
8. An onset-suppressing and/or therapeutic agent for insulin resistance syndrome, which comprises a pharmaceutically acceptable anion exchange resin as an active ingredient.
9. The onset-suppressing and/or therapeutic agent according to the aforementioned 8, wherein the pharmaceutically acceptable anion exchange resin has a bile acid-adsorbing ability.
10. The onset-suppressing and/or therapeutic agent according to the aforementioned 8 or 9, wherein the pharmaceutically acceptable anion exchange resin is selected from colestimide, cholestyramine resin, colestipol, sevelamer hydrochloride, and colesevelam hydrochloride.
11. The onset-suppressing and/or therapeutic agent according to the aforementioned 8 or 9, wherein the pharmaceutically acceptable anion exchange resin is an anion exchange resin synthesized by a polymerization reaction of an epichlorohydrin derivative and an amine of which typical examples are imidazole derivatives.
12. The onset-suppressing and/or therapeutic agent according to any one of the aforementioned 8 to 11, wherein the pharmaceutically acceptable anion exchange resin is colestimide.
13. The onset-suppressing and/or therapeutic agent according to any one of the aforementioned 8 to 12, with which an oral hypoglycemic agent is used simultaneously, separately, or successively.
14. The onset-suppressing and/or therapeutic agent according to 13, wherein the oral hypoglycemic agent is selected from the group consisting of $\alpha$-glucosidase inhibitors, biguanides, insulin sensitivity improving agents, sulfonylurea agents, rapid-acting insulin secretagogues, pharmaceutical preparations comprising GLP-1 or derivatives thereof, and DPP-IV inhibitors.
15. A prophylactic, improving and/or therapeutic agent for a disease or symptom resulting from insulin resistance, which comprising a pharmaceutically acceptable anion exchange resin as an active ingredient.
16. The prophylactic, improving and/or therapeutic agent according to the aforementioned 15, wherein the disease or symptom resulting from insulin resistance is selected from the group consisting of hyperinsulinism, abnormal lipid metabolism, arteriosclerosis, abnormal vascular endothelial function, coronary artery disease, cardiovascular disease, renal dysfunction, hypertension, fatty liver, type 2 diabetes, hyperuricemia, multiple risk factor syndrome, and gestational diabetes.
17. The prophylactic, improving and/or therapeutic agent according to the aforementioned 15, wherein the disease or symptom resulting from insulin resistance is selected from the group consisting of hyperinsulinism, abnormal lipid metabolism, abnormal vascular endothelial function, coronary artery disease, cardiovascular disease, renal dysfunction, hypertension, fatty liver, type 2 diabetes, and hyperuricemia.
18. The prophylactic, improving and/or therapeutic agent according to the aforementioned 15, wherein the disease or symptom resulting from insulin resistance is selected from the group consisting of hyperinsulinism, abnormal lipid metabolism, renal dysfunction, fatty liver, type 2 diabetes, and hyperuricemia.
19. The prophylactic, improving and/or therapeutic agent according to the aforementioned 16 or 17, wherein the coronary artery disease or cardiovascular disease is myocardial infarction, cerebral infarction, or cerebral apoplexy.
20. The prophylactic, improving and/or therapeutic agent according to the aforementioned 16, wherein the multiple risk factor syndrome is syndrome X, visceral fat syndrome, or metabolic syndrome.
21. The prophylactic, improving and/or therapeutic agent according to any one of the aforementioned 15 to 20, wherein the pharmaceutically acceptable anion exchange resin has a bile acid adsorbing ability.
22. The prophylactic, improving and/or therapeutic agent according to any one of the aforementioned 15 to 21, wherein the pharmaceutically acceptable anion exchange resin is selected from the group consisting of colestimide, cholestyramine resin, colestipol, sevelamer hydrochloride, and colesevelam hydrochloride.
23. The prophylactic, improving and/or therapeutic agent according to any one of the aforementioned 15 to 21, wherein the pharmaceutically acceptable anion exchange resin is an anion exchange resin synthesized by a polymerization reaction of an epichlorohydrin derivative and an amine of which typical examples are imidazole derivatives.

24. The prophylactic, improving and/or therapeutic agent according to any one of the aforementioned 15 to 23, wherein the pharmaceutically acceptable anion exchange resin is colestimide.

25. The prophylactic, improving and/or therapeutic agent according to any one of the aforementioned 15 to 24, with which an oral hypoglycemic agent is used simultaneously, separately or at different timings.

26. The prophylactic, improving and/or therapeutic agent according to the aforementioned 25, wherein the oral hypoglycemic agent is selected from α-glucosidase inhibitors, biguanides, insulin sensitivity improving agents, sulfonylurea agents, rapid-acting insulin secretagogues, pharmaceutical preparations comprising GLP-1 or derivatives thereof and DPP-IV inhibitors.

Effect of the Invention

According to the present invention, a medicament can be provided which exhibits an insulin resistance-improving action regardless of a volume of meal or glucose absorption from the gastrointestinal tract .

Brief Description of the Drawings

[Fig. 1] Drawing which depicts changes in body weight in Example 1.

[Fig. 2] Drawing which depicts changes in feed intake in Example 1.

[Fig. 3] Drawings which depicts comparison between the control group and the colestimide prophylactic treatment group for average feed intake in the 1st to 12th weeks of the test period in Example 1 (Fig. 3-1) and comparison across the control group, the colestimide prophylactic treatment group, and the colestimide therapeutic treatment group for average feed intake in the 12th to 20th weeks of the test period (Fig. 3-2).

[Fig. 4] Drawing which depicts changes in plasma blood glucose levels in Example 1.

[Fig. 5] Drawing which depicts changes in plasma insulin levels in Example 1.

[Fig. 6] Drawing which depicts the insulin sensitivity indices (insulin resistance-improving action) in Example 1.

[Fig. 7] Drawing which depicts glucose uptake into peripheral tissues associated with the insulin resistance-improving action in Example 1.

[Fig. 8] Drawings which depicts fasting blood glucose levels in the control group and the colestimide group (Fig. 8-1), fasting insulin levels in the colestimide group (Fig. 8-2) and blood glucose levelAUCs (0 to 120 min) in the colestimide group (Fig. 8-3) in Example 2.

[Fig. 9] Drawings which depicts fasting blood glucose levels in the control group and the colesevelam hydrochloride group (Fig. 9-1), fasting insulin levels in the colesevelam hydrochloride group (Fig. 9-2), blood glucose level AUCs (0 to 120 min) in the colesevelam hydrochloride group (Fig. 9-3) in Example 3.

[Fig. 10] Drawings which depicts fasting blood glucose levels in the control group and the sevelamer hydrochloride group (Fig. 10-1), fasting insulin levels in the sevelamer hydrochloride group (Fig. 10-2) and blood glucose levelAUCs (0 to 120 min) in the sevelamer hydrochloride group (Fig. 10-3) in Example 4. Best Mode for Carrying out the Invention

Hereafter, the present invention will be explained in more detail.

In the present invention, a pharmaceutically acceptable anion exchange resin means an anion exchange resin that can be administered as a medicament, and preferred examples thereof include anion exchange resins having a bile acid-adsorbing ability. The anion exchange resins are not particularly limited so long as they exhibit an insulin resistance-improving action when administered to hyperlipemia pathologic models such as explained in the examples mentioned later.

An example includes colestimide (2-methylimidazole-epichlorohydrin copolymers) which is the most preferred example. Although colestimide has an irregular and disordered complicated three-dimensional structure, the resin is represented by the basic structure of the following formula (I), of which structure is partially represented by the following formula (II), and can be obtained by a polymerization reaction of an epichlorohydrin derivative and an amine of which typical example is an imidazole derivatives, specifically, by the preparation method described in Japanese Patent Unexamined Publication (Kokai) No. 60-209523.

[Formula 1]

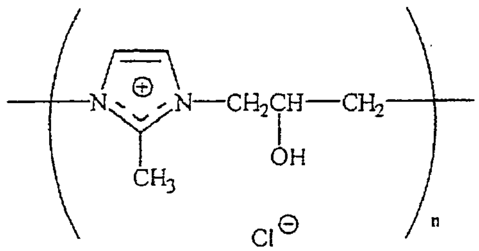

· · · (I)

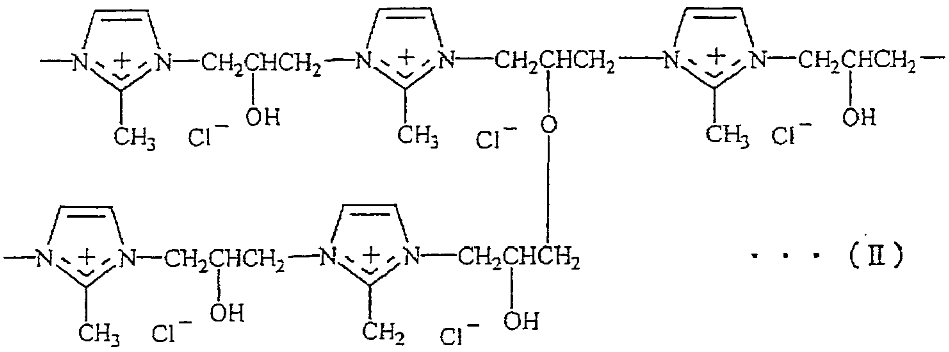

· · · (II)

Colestimide is registered with a nonproprietary name of colestimide (chemical name 2-methylimidazole-epichlorohydrin copolymer) in JAN. The resin is registered with a nonproprietary name of colestilan (chemical name: 2-methylimidazole polymer with 1-chloro-2,3-epoxypropane) in INN.

Other preferred examples of the anion exchange resin include the aforementioned cholestyramine resin, colestipol (N-(2-aminoethyl-N'-[2-[(2-aminoethyl)amino]ethyl]-1,2-ethanediamine polymer added with (chloromethyl)oxylane) and the like. These resins are sold by Sigma. The cholestyramine resin is a strong basic anion exchange resin containing a styrene-divinylbenzene copolymer added with quaternary ammonium groups, and the basic structure thereof is represented by the following formula (III).

[Formula 2]

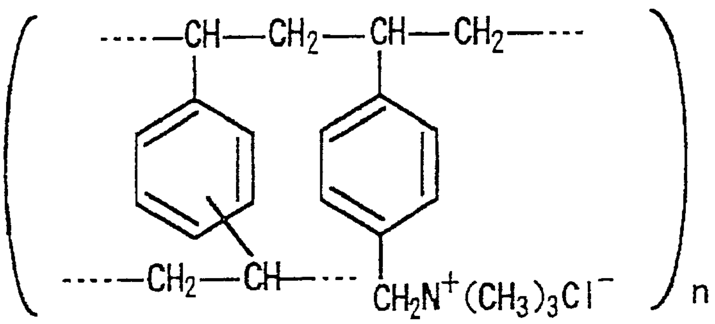

······· (III)

Further, the basic structure of sevelamer hydrochloride is represented by the following formula, and said resin can be prepared by the method described in U.S. Patent No. 5,496,545 or similar methods.

[Formula 3]

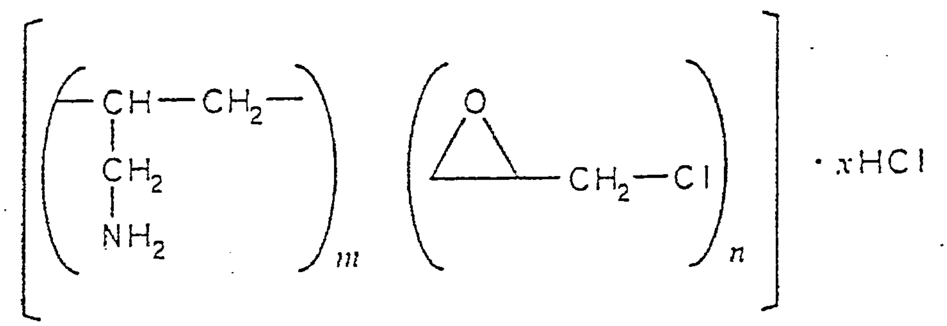

The basic structure of colesevelam hydrochloride is represented by the following formula. The resin can be prepared by the method described in U.S. Patent No. 5,607,669 or similar methods.

[Formula 4]

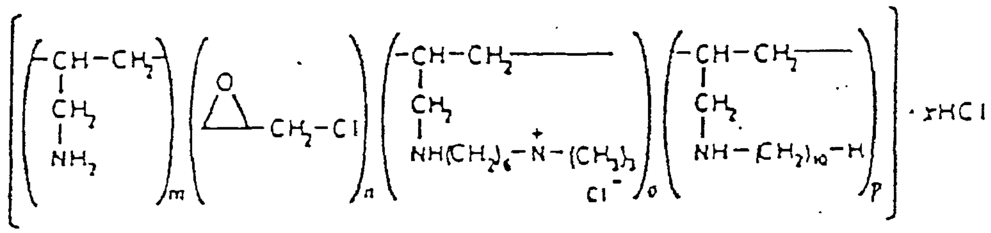

In addition, the anion exchange resins described in International Patent Unexamined Publication in Japanese (Kohyo) Nos. 9-504782, 9-500368, 10-501264, 10-501842, 11-507093, 11-5120 74, and 5-512332 and Japanese Patent Unexamined Publication (Kokai) Nos. 8-208 750, 9-202732, 10-114661, and 11-228449 can also be used in the present invention so long as they do not depart from the gist of the present invention.

As the insulin resistance-improving agent of the present invention, the aforementioned compounds as an active ingredient per se may be used. Pharmaceutical compositions containing the aforementioned active ingredients and commonly used additives for pharmaceutical preparations are preferably prepared and used.

Examples of such pharmaceutical compositions include tablets, capsules, subtilized granules, pills, troches, solutions and the like, and these are orally administered (including sublingual administration).

Oral pharmaceutical compositions can be prepared by conventional methods such as mixing, filling and compressing. Further, the active ingredient may be distributed in a pharmaceutical composition by using a large amount of excipient and by applying repeated mixing operations. For example, tablets or capsules used for oral administration are preferably provided as unit administration products and may contain carriers ordinarily used for pharmaceutical preparations such as binders, fillers, diluents, compressing agents, lubricants, disintegrating agents, coloring materials, flavors, and wetting agents. Tablets may be prepared as, for example, coated tablets by using a coating agent according to methods known to those skilled in the art.

Preferred examples of the excipients include cellulose, mannitol, lactose and the like. Starch, polyvinylpyrrolidone, starch derivatives such as sodium starch glycolate and the like as disintegrating agents, sodium laurylsulfate as lubricant, and the like can be used as additives for pharmaceutical preparations. Examples of pharmaceutical compositions in the form of oral liquid include pharmaceutical compositions such as aqueous or oily suspensions, solutions, emulsions, syrups and elixirs and dry pharmaceutical compositions that can be redissolved in water or a suitable medium before use.

The above solutions may be mixed with ordinary additives, for example, suspending agents such as sorbitol, syrup, methylcellulose, gelatin, hydroxyethylcellulose, carboxymethylcellulose, aluminum stearate gel and hydrogenated

edible fat; emulsifiers such as lecithin, sorbitan monooleate and gum arabic; oily esters such as almond oil, rectified coconut oil and glycerin esters; nonaqueous media such as propylene glycol and ethyl alcohol (edible oil may be included); preservatives such as methyl ester, ethyl ester or propyl ester of p-hydroxybenzoic acid and sorbic acid; usual flavors or coloring materials and the like, if necessary.

Where the aforementioned oral pharmaceutical composition is in the form of, for example, a tablet, capsule, subtilized granule or the like, the composition usually contains 5 to 95% by weight, preferably 25 to 90% by weight, of the active ingredient.

Colestimide is sold by Mitsubishi Pharma Corporation with a trade name of Cholebine. Cholebine, per se, may be used according to the present invention. Further, sevelamer hydrochloride is sold by Chugai Pharmaceutical Co., Ltd. and Genzyme Corporation with a trade name of Renagel and by Kirin Brewery Co., Ltd. with a trade name of Phosblock. Renagel, per se, may be used according to the present invention. Further, colesevelam hydrochloride is marketed by Sankyo Pharma Inc. with a trade name of WelChol. WelChol, per se, may be used according to the present invention.

A dose of the insulin resistance-improving agent of the present invention can be suitably selected depending on the active ingredient to be used, the age, health condition and body weight of a patient, severity of diseases, type and frequency of treatment simultaneously applied, nature of desired effect, and the like. In general, as for colestimide as an example, a daily dose of 1 to 60 g for an adult in terms of the amount of the active ingredient can be administered once or several times per day.

According to the present invention, the aforementioned pharmaceutically acceptable anion exchange resin and another oral hypoglycemic agent can be used simultaneously, separately, or successively. Specifically, a medicament comprising the aforementioned pharmaceutically acceptable anion exchange resin as an active ingredient and another oral hypoglycemic agent can be administered as a single pharmaceutical composition or as separate pharmaceutical compositions prepared on the basis of doses determined by suitable increase or decrease depending on the age, condition, sexuality, symptoms of a patient and the like. Where separate pharmaceutical compositions are administered, they can be administered in the same or different dosage forms simultaneously, in the same or different dosage forms at different times on the same day, or with given intervals over several days, several weeks or several months depending on the age, condition, sexuality, symptoms of a patient and the like.

Examples of the oral hypoglycemic agent used in the present invention include α-glucosidase inhibitors, biguanides, insulin sensitivity improving agents, sulfonylurea agents, rapid-acting insulin secretagogues and the like, but not limited to these examples. The α-glucosidase inhibitors are not particularly limited, and examples thereof include acarbose, voglibose and the like. The biguanides are not particularly limited, and examples thereof include metformin and the like. The insulin sensitivity improving agents are not particularly limited, and examples thereof include pioglitazone and the like. The sulfonylurea agents are not particularly limited, and examples thereof include gliclazide, glibenclamide, glimepide and the like. The insulin secretagogues are not particularly limited, and examples thereof include nateglinide and the like. As these oral hypoglycemic agents, those soled as reagents may be used or those that have already been launched as medicaments may be used.

In the above descriptions, embodiments as an insulin resistance-improving agent are mainly explained. However, the agent of the present invention can be used in the same manner for embodiments as an onset-suppressing and/or therapeutic agent for insulin resistance syndromes and prophylactic, improving and/or therapeutic agent for diseases and symptoms resulting from insulin resistance.

Insulin resistance syndrome was advocated by De Frozo in 1991 and is thought to be one of the onset mechanisms of diabetes. The resistance results in a risk of hyperlipemia and hypertension as well as diabetes. Obesity is considered to be a cause of the resistance. The resistance is also considered to be a direct cause of arteriosclerosis.

Specific examples of the diseases and symptoms resulting from insulin resistance include hyperinsulinism, abnormal lipid metabolism, arteriosclerosis, abnormal vascular endothelial function, coronary artery diseases, cardiovascular diseases, renal dysfunction, hypertension, fatty liver, type 2 diabetes, hyperuricemia, multiple risk factor syndromes, gestational diabetes, and the like. Examples of the coronary artery diseases and cardiovascular diseases include myocardial infarction, cerebral infarction, cerebral apoplexy, and the like. Further, examples of the multiple risk factor syndromes include syndrome X advocated by Reaven in 1988, visceral fat syndrome advocated by Matsuzawa et al. in 1987, deadly quartet advocated by Kaplan in 1989, metabolic syndrome advocated by the National Cholesterol Education Program (NCEP) ATP-III (Adult Treatment Panel III) in the U.S. in 2001, and the like.

## Examples

The present invention will be explained more specifically with reference to the following examples. However, the scope of the present invention is not limited to these examples. The colestimide used in the following examples was prepared according to the method described in Japanese Patent Unexamined Publication (Kokai) No. 60-209523. Further, sevelamer hydrochloride and colesevelam hydrochloride used were appropriately prepared from sold products.

Example 1

1. Test method

ApoE3 Leiden mice (male, TNO Pharma, Leiden, Netherlands) (n = 45) were fed with a high fat diet (45.4% fat) for 3 weeks and divided into two groups based on body weight and serum parameters (total cholesterol (TC), triglyceride level (TG) and glucose level (Glc)). One group (n = 30) was continuously fed with the high fat diet (high fat diet group), and the other group (n = 15) with the high fat diet containing 1.5% (w/w) colestimide (colestimide prophylactic treatment group).

Twelve weeks later, the high fat diet group was further divided into two groups based on body weight and the aforementioned serum parameters (TC, TG, Glc). One group was fed with the high fat diet (control group, n = 15), and the other group with the high fat diet containing 1.5% (w/w) colestimide (colestimide therapeutic treatment group, n = 15).

Eight weeks later, insulin resistance was measured by the hyperinsulinemic clamp method. An indwelling needle was inserted into the caudal vein of each animal under anesthesia (0.5 ml/kg Hypnorm and 12.5 mg/g midazalom), and insulin was infused to achieve a high insulin level (hyperinsulinemic state). To maintain the blood glucose level lowered by the insulin infusion at 7.0 mM, a 12.5°/ D-glucose solution was continuously infused with controlling the infusion rate.
The insulin sensitivity index was represented in terms of infusion amount of the 12.5% D-glucose solution (glucose infusion rate) required to maintain the blood glucose level at 7.0 mM Better insulin sensitivity increases infusion amount of D-glucose.

$$\text{Insulin sensitivity index} = \text{glucose infusion rate (mmol glucose/min/kg)}$$

2. Results

(1) Body weight

Changes in body weight in the control group (+), the colestimide prophylactic treatment group (o) and the colestimide therapeutic treatment group (▲) are shown in Fig. 1. Body weights in the colestimide prophylactic treatment group (20-week treatment) were significantly lower than those in the control group ($p < 0.05$). Further, body weights in the colestimide therapeutic treatment group (8-week treatment from the 12th week) were not significantly different from, but always lower than, those in the control group.

(2) Feed intake

Changes in feed intake in the control group (+), the colestimide prophylactic treatment group (o) and the colestimide therapeutic treatment group (▲) are shown in Fig. 2. The feed intakes in the colestimide prophylactic treatment group were always higher from the 1st week of the treatment than those in the control group ($p < 0.05$). It can be understood that the feed intakes in the colestimide therapeutic treatment group were not significantly different from those in the control group, but more increased within a short period of time after the treatment with colestimide as compared with the control group.

Comparison of the average feed intake during the 1st to 12th weeks of the test period is shown in Fig. 3-1, and comparison of the average feed intake during the 12th to 20th weeks of the test period is shown in Fig. 3-2. In the bar graph of Fig. 3-1, the left bar represents the result for the control group, and the right bar for the colestimide prophylactic treatment group. In the bar graph of Fig. 3-2, the left bar represents the result for the control group, the middle bar for the colestimide prophylactic treatment group, and the right bar for the colestimide therapeutic treatment group. The average feed intakes in the colestimide prophylactic treatment group and the colestimide therapeutic treatment group significantly increased as compared with those in the control group ($p < 0.05$).

From the results shown in Figs. 3-1, 3-2 and 1, it was revealed that lower body weights were observed in the colestimide prophylactic treatment group as compared with the control group in spite of the increase in feed intake.

(3) Plasma sugar levels (glucose levels)

Changes in the plasma glucose level in the control group (+), the colestimide prophylactic treatment group (o) and the colestimide therapeutic treatment group (A) are shown in Fig. 4. The blood glucose levels significantly decreased in the colestimide prophylactic treatment group from the 2nd to 12th weeks, and in the colestimide prophylactic treatment

group and the colestimide therapeutic treatment group in the 16th week as compared with those in the control group ($p < 0.05$).

(4) Plasma insulin levels

Changes in the plasma insulin level in the control group (+), the colestimide prophylactic treatment group (o) and the colestimide therapeutic treatment group (▲) are shown in Fig. 5. In the bar graph of Fig. 5, the left bar represents the result for the control group, the middle bar for the colestimide prophylactic treatment group, and the right bar for the colestimide therapeutic treatment group. The insulin levels in the control group markedly increased after 16 weeks of the high fat diet loading. This result suggests that the mice were insulin resistant at that time. The insulin levels significantly decreased in both of the colestimide prophylactic treatment group and the colestimide therapeutic treatment group ($p < 0.05$).

(5) Insulin sensitivity indices (insulin resistance-improving action)

The results are shown in Fig. 6. In the bar graph of Fig. 6, the left bar represents the result for the control group, the middle bar for the colestimide prophylactic treatment group, and the right bar for the colestimide therapeutic treatment group. The insulin sensitivity indices significantly increased in both of the colestimide prophylactic treatment group and the colestimide therapeutic treatment group as compared with those in the control group ($p < 0.05$), indicating improvement of insulin sensitivity.

(6) Glucose uptake into tissues associated with insulin resistance improvement

The results are shown in Fig. 7. In the bar graph of Fig. 7, the left bar represents the result for the control group, the middle bar for the colestimide prophylactic treatment group, and the right bar for the colestimide therapeutic treatment group. No significant difference was observed for all of the groups in glucose uptake into tissues under the condition before insulin infusion (basal), whist glucose uptakes into the peripheral tissues (skeletal muscles) in the hyperinsulinemic state significantly increased in the colestimide prophylactic treatment group and the colestimide therapeutic treatment group as compared with those in the control group ($p < 0.05$). This result indicates that glucose uptake into the peripheral tissues increased only in the presence of insulin, and thus improvement of insulin sensitivity was demonstrated.

From the above results, it is clearly understood that colestimide exhibits an insulin resistance-improving action, and this action is not resulting from suppression of feed intake or glucose absorption from the gastrointestinal tract.

Example 2

1. Test method

KKAy mice (male, Clea Japan, Inc., n = 8) were used. The control group was fed with a high fat diet (23.6% fat), and the colestimide group was fed with the high fat diet containing 2% colestimide. A glucose tolerance test was performed according to a conventional method after 2 weeks of the treatment. The mice were fasted overnight, and blood was collected before loading of glucose. Then, a glucose solution was orally given, and blood glucose levels were measured after 30, 60, 90 and 120 minutes. The blood glucose level AUCs (0 to 120 min) were calculated by using the blood glucose levels obtained. The fasting blood glucose levels and the fasting insulin levels were measured by using the blood samples collected before the loading of glucose.

2. Results

(1) Fasting blood glucose levels

Fasting blood glucose levels in the control group and the colestimide group are shown in Fig. 8-1. The fasting blood glucose levels in the colestimide group significantly decreased as compared with those in the control group ($p < 0.01$).

(2) Fasting insulin levels

Fasting insulin levels in the control group and the colestimide group are shown in Fig. 8-2. The fasting insulin levels in the colestimide group significantly decreased as compared with those in the control group ($p < 0.01$).

(3) Blood glucose levelAUCs (0 to 120 min)

Blood glucose level AUCs (0 to 120 min) in the control group and the colestimide group are shown in Fig. 8-3. The blood glucose level AUCs (0 to 120 min) in the colestimide group significantly decreased as compared with those in the control group ($p < 0.01$).

The above results suggested that colestimide had an insulin resistance-improving action.

Example 3

1. Test method

KKAy mice (male, Clea Japan, Inc., n = 8) were used. The control group was fed with a high fat diet (23.6% fat), and the colesevelam hydrochloride group was fed with the high fat diet containing 2% colesevelam hydrochloride. A glucose tolerance test was performed according to a conventional method after 2 weeks of the treatment. The mice were fasted overnight, and blood was collected before loading of glucose. Then, a glucose solution was orally given, and blood glucose levels were measured after 30, 60, 90 and 120 minutes. The blood glucose level AUCs (0 to 120 min) were calculated by using the blood glucose levels obtained. The fasting blood glucose levels and the fasting insulin levels were measured by using the blood samples collected before the loading of glucose.

2. Results

(1) Fasting blood glucose levels

Fasting blood glucose levels in the control group and the colesevelam hydrochloride group are shown in Fig. 9-1. The fasting blood glucose levels in the colesevelam hydrochloride group significantly decreased as compared with those in the control group ($p < 0.01$).

(2) Fasting insulin levels

Fasting insulin levels in the control group and the colesevelam hydrochloride group are shown in Fig. 9-2. The fasting insulin levels in the colesevelam hydrochloride group significantly decreased as compared with those in the control group ($p < 0.01$).

(3) Blood glucose level AUCs (0 to 120 min)

Blood glucose level AUCs (0 to 120 min) in the control group and the colesevelam hydrochloride group are shown in Fig. 9-3. The blood glucose level AUCs (0 to 120 min) in the colesevelam hydrochloride group significantly decreased as compared with those in the control group ($p < 0.01$).

The above results suggested that colesevelam hydrochloride had an insulin resistance-improving action.

Example 4

1. Test method

KKAy mice (male, Clea Japan, Inc., n = 8) were used. The control group was fed with a high fat diet (23.6% fat), and the sevelamer hydrochloride group was fed with the high fat diet containing 2% sevelamer hydrochloride. A glucose tolerance test was performed according to a conventional method after 2 weeks of the treatment. The mice were fasted overnight, and blood was collected before loading of glucose. Then, a glucose solution was orally given, and blood glucose levels were measured after 30, 60, 90 and 120 minutes. The blood glucose level AUCs (0 to 120 min) were calculated by using the obtained blood glucose levels. The fasting blood glucose levels and the fasting insulin levels were measured by using the blood samples collected before the loading of glucose.

2. Results

(1) Fasting blood glucose levels

Fasting blood glucose levels in the control group and the sevelamer hydrochloride group are shown in Fig. 10-1. The fasting blood glucose levels in the colesevelam hydrochloride group significantly decreased as compared with

those in the control group ($p < 0.05$).

(2) Fasting insulin levels

Fasting insulin levels in the control group and the sevelamer hydrochloride group are shown in Fig. 10-2. The fasting insulin levels in the sevelamer hydrochloride group significantly decreased as compared with those in the control group ($p < 0.01$).

(3) Blood glucose levelAUCs (0-120 min)

Blood glucose levelAUCs (0 to 120 min) in the control group and the sevelamer hydrochloride group are shown in Fig. 10-3. The blood glucose levelAUCs (0 to 120 min) in the sevelamer hydrochloride group significantly decreased as compared with those in the control group ($p < 0.01$).

The above results suggested that sevelamer hydrochloride had an insulin resistance-improving action.

Industrial Applicability

According to the present invention, a medicament can be provided which exhibits an insulin resistance-improving action regardless of volume of meal or glucose absorption from the gastrointestinal tract.
The present application is based on a Japanese patent application filed on March 26, 2004 (Japanese Patent Application No. 2004-091508), of which entire disclosures are incorporated herein by reference.

## Claims

1. An agent for prophylactic, improving and/or therapeutic treatment of a disease or symptom resulting from insulin resistance, which comprises a pharmaceutically acceptable anion exchange resin as an active ingredient, wherein the disease or symptom resulting from insulin resistance is selected from the group consisting of abnormal lipid metabolism, arteriosclerosis, abnormal vascular endothelial function, coronary artery disease, cardiovascular disease, renal dysfunction, hypertension, type 2 diabetes, hyperuricemia, multiple risk factor syndrome, and gestational diabetes.

2. The prophylactic, improving and/or therapeutic agent according to claim 1. wherein the pharmaceutically acceptable anion exchange resin has a bile acid adsorbing ability.

3. The prophylactic, improving and/or therapeutic agent according to any one of claims 1 to 2, wherein the pharmaceutically acceptable anion exchange resin is selected from the group consisting of colestimide, cholestyramine resin, colestipol, sevelamer hydrochloride, and colesevelam hydrochloride.

4. The prophylactic, improving and/or therapeutic agent according to any one of claims 1 to 3, wherein the pharmaceutically acceptable anion exchange resin is an anion exchange resin synthesized by a polymerization reaction of an epichlorohydrin derivative and an amine of which typical example includes an imidazole derivative.

5. The prophylactic, improving and/or therapeutic agent according to any one of claims 1 to 4, wherein the pharmaceutically acceptable anion exchange resin is colestimide.

6. The prophylactic, improving and/or therapeutic agent according to any one of claims 1 to 5, with which an oral hypoglycemic agent is used simultaneously, separately, or successively.

7. The prophylactic, improving and/or therapeutic agent according to claim 6, wherein the oral hypoglycemic agent is selected from the group consisting of $\alpha$-glucosidase inhibitors, biguanides, insulin sensitivity improving agents, sulfonylurea agents, rapid-acting insulin secretagogues, pharmaceutical preparations comprising GLP-1 or derivatives thereof, and DPP-IV inhibitors.

Fig. 1

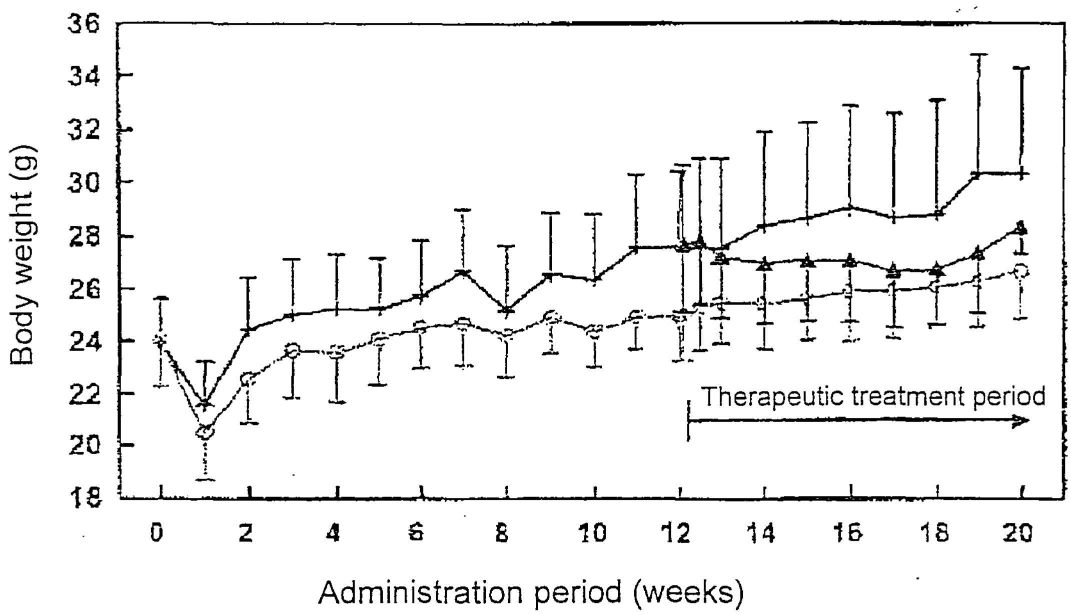

36
34
32
30
28
26
24
22
20
18
Body weight (g)
0
2
4
6
8
10
12
14
16
18
20
Therapeutic treatment period
Administration period (weeks)


Fig. 2

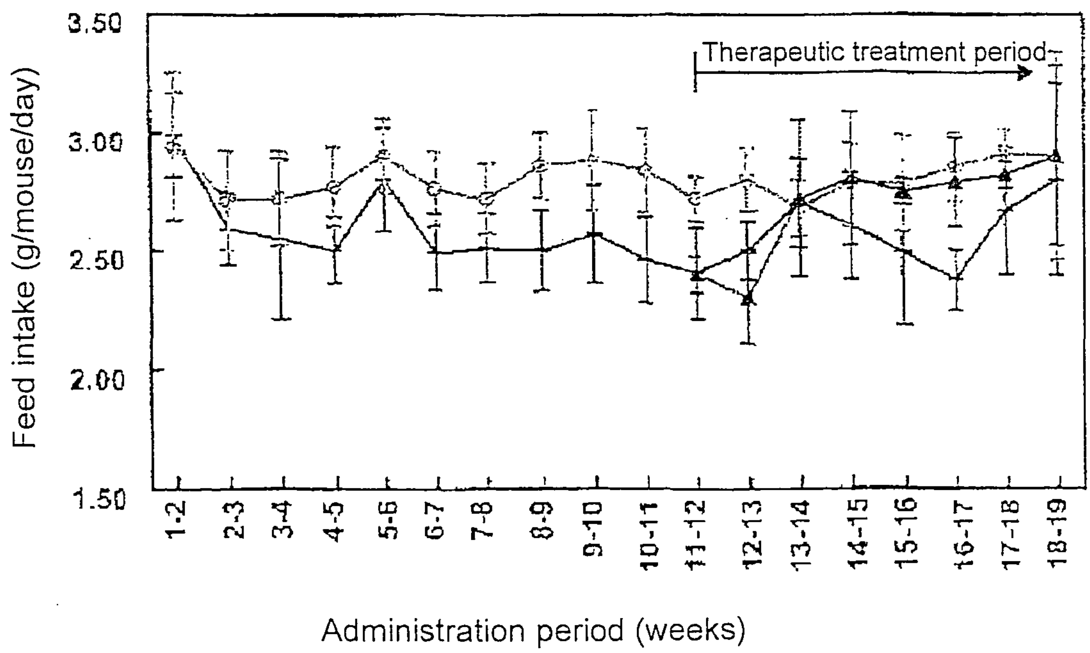

3.50
3.00
2.50
2.00
1.50
Feed intake (g/mouse/day)
Therapeutic treatment period
1-2
2-3
3-4
4-5
5-6
6-7
7-8
8-9
9-10
10-11
11-12
12-13
13-14
14-15
15-16
16-17
17-18
18-19
Administration period (weeks)

Fig. 3

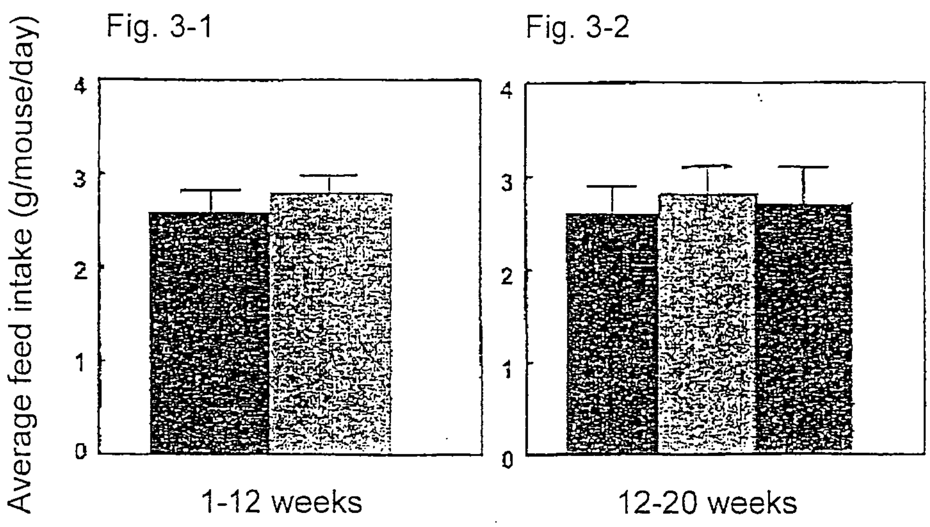
Fig. 3-1
Fig. 3-2
Average feed intake (g/mouse/day)
4
3
2
1
0
1-12 weeks
12-20 weeks

Fig. 4

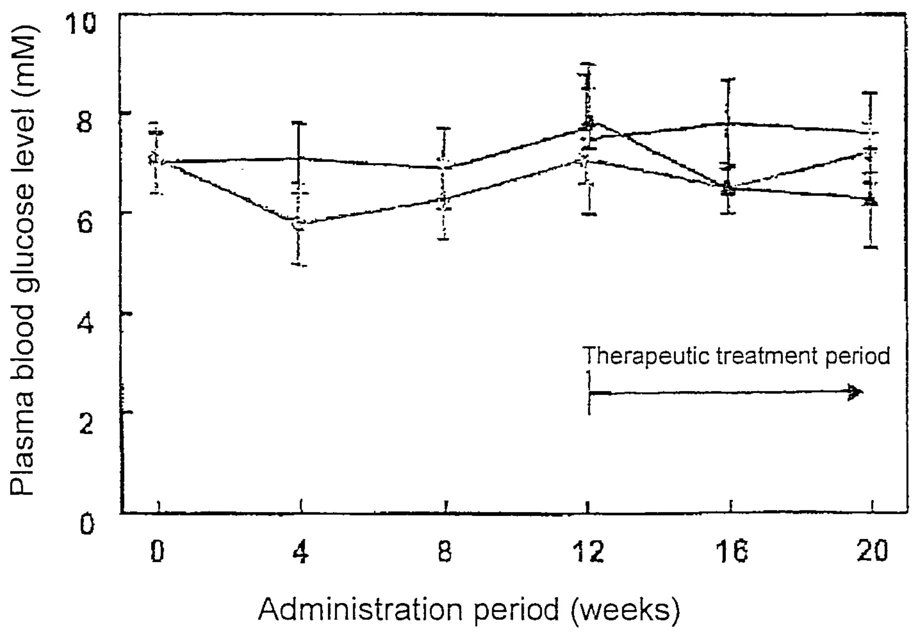
Plasma blood glucose level (mM)
10
8
6
4
2
0
Therapeutic treatment period
0
4
8
12
16
20
Administration period (weeks)

Fig. 5

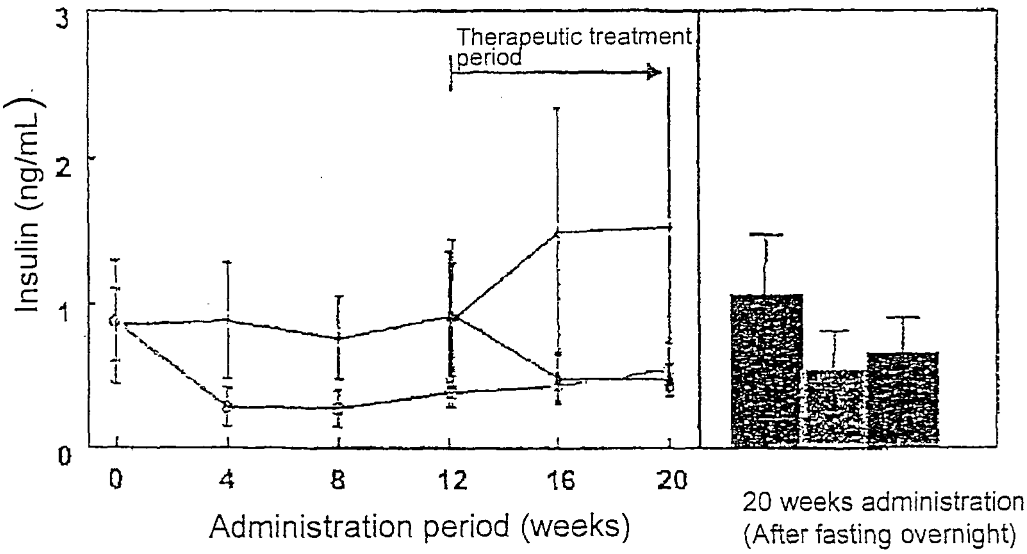
3
2
1
0
Insulin (ng/mL)
Therapeutic treatment period
0
4
8
12
16
20
Administration period (weeks)
20 weeks administration
(After fasting overnight)

Fig. 6

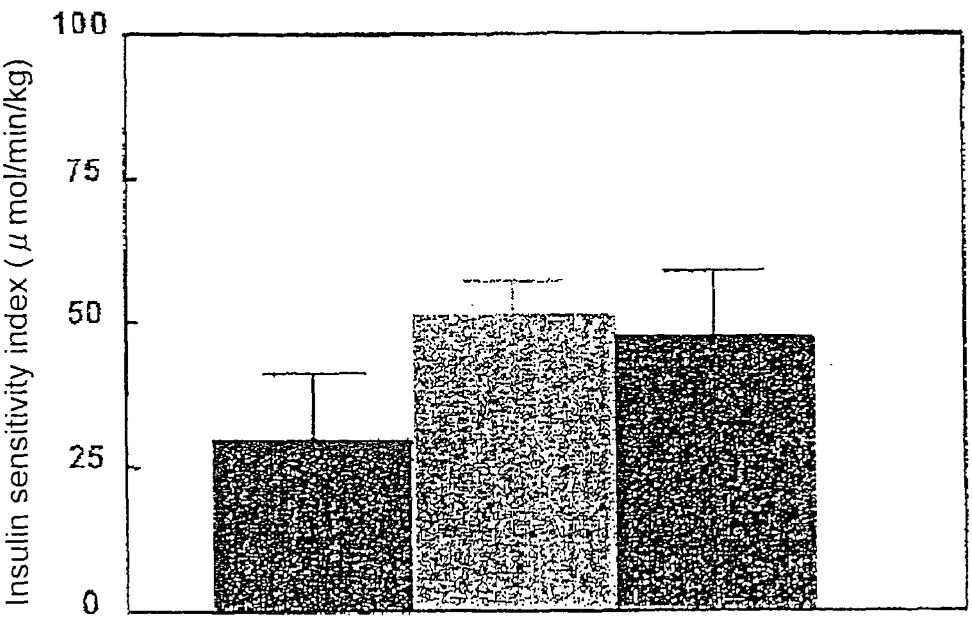
100
75
50
25
0
Insulin sensitivity index ( μ mol/min/kg)

Fig. 7

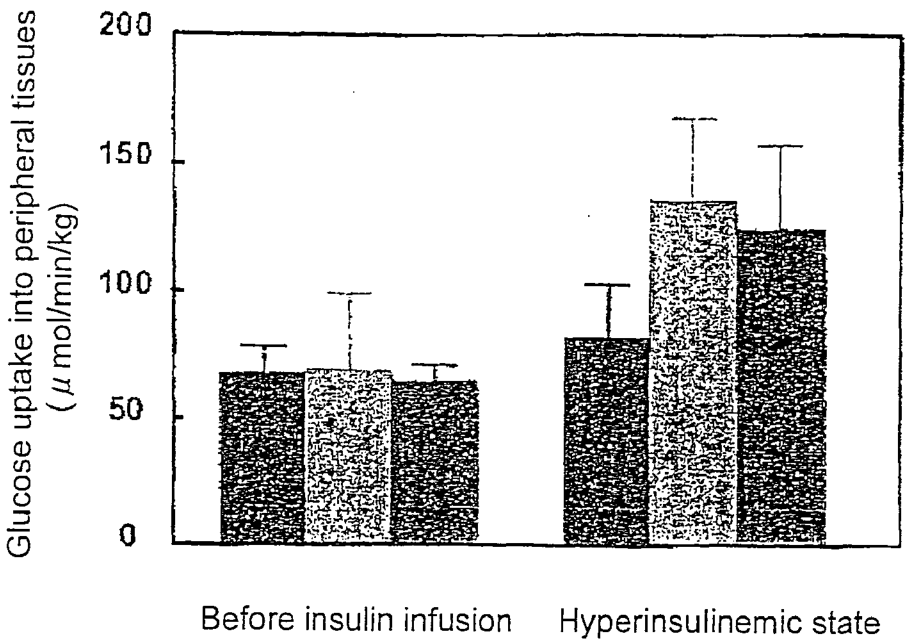
Glucose uptake into peripheral tissues
( μ mol/min/kg)
200
150
100
50
0
Before insulin infusion
Hyperinsulinemic state

Fig. 8

Fig. 8-1

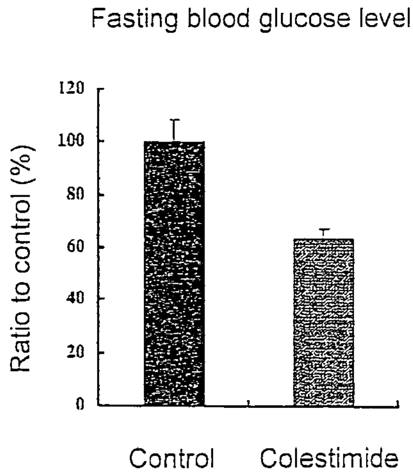


Fig. 8-2

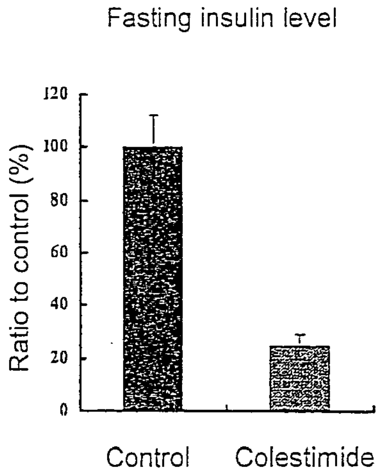


Fig. 8-3

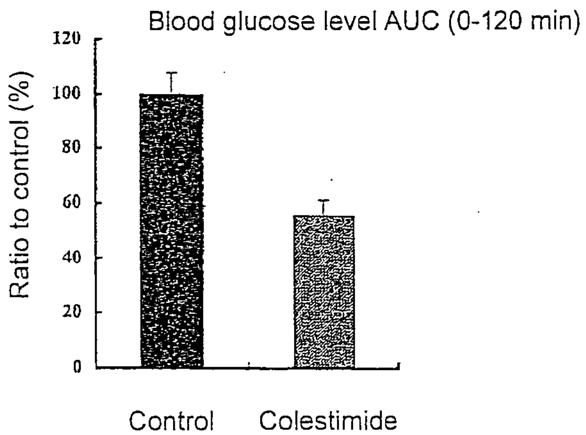

Fig. 9

Fig. 9-1

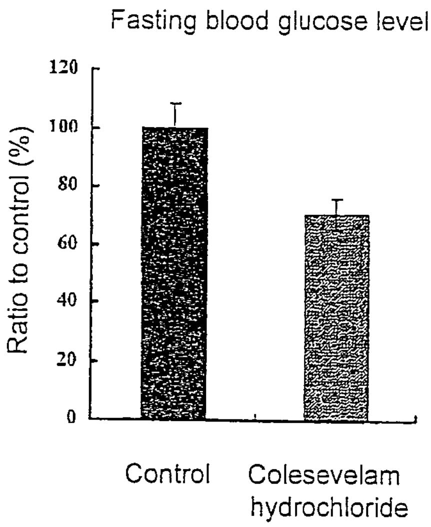


Fig. 9-2

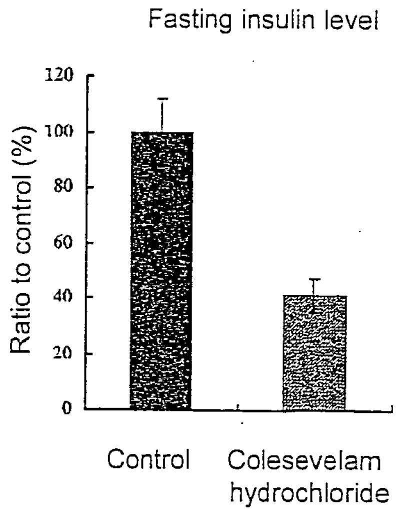


Fig. 9-3

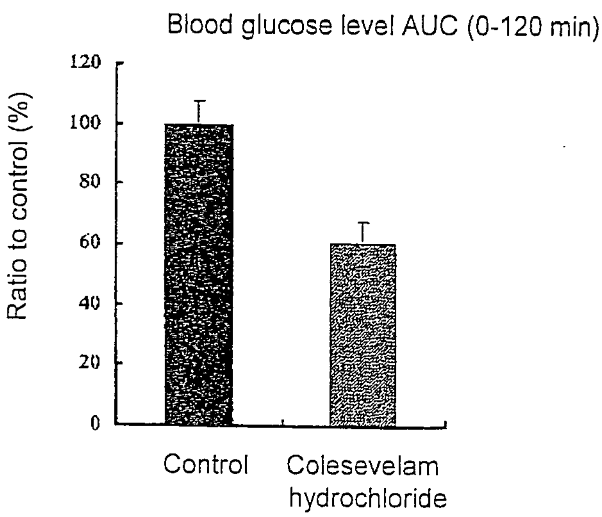

Fig. 10

Fig. 10-1

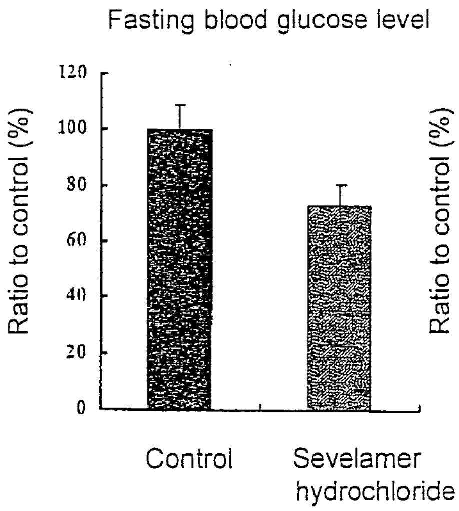


Fig. 10-2

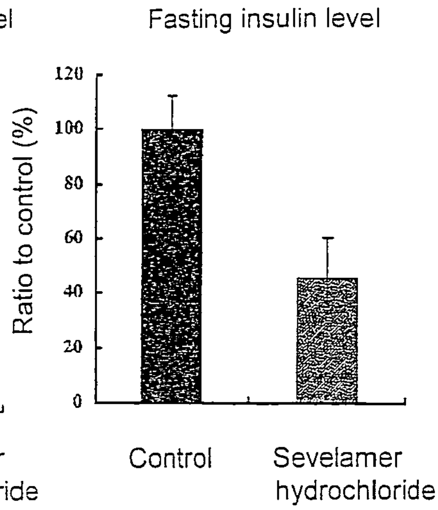


Fig. 10-3

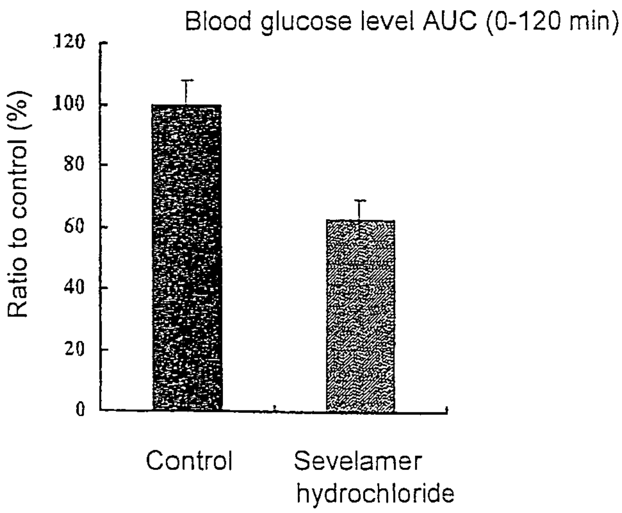

Europäisches Patentamt
European Patent Office
Office européen des brevets

# EUROPEAN SEARCH REPORT

Application Number
EP 11 00 2551

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/011308 A1 (MITSUBISHI PHARMA CORP [JP]; OBA KENZO [JP]) 13 February 2003 (2003-02-13) * page 2, paragraph 4-8 * * page 5; example 1 * * claims 1-23 * ----- | 1-7 | INV. A61K31/785 A61K31/74 A61K31/787 A61K45/00 A61P3/06 A61P3/10 A61P5/50 A61P9/00 A61P9/10 A61P9/12 A61P13/12 |
| X | GARG A ET AL: "Cholestyramine Therapy for Dyslipidemia in Non-Insulin-dependent Diabetes Mellitus", ANNALS OF INTERNAL MEDICINE, NEW YORK, NY; US, US, vol. 121, no. 6, 15 September 1994 (1994-09-15), pages 416-422, XP002989475, ISSN: 0003-4819 * the whole document * ----- | 1-7 | |
| X | US 5 298 497 A (TSCHOLLAR WERNER [US] ET AL) 29 March 1994 (1994-03-29) * column 19; examples 15,16 * ----- | 1-7 | TECHNICAL FIELDS SEARCHED (IPC) |
| X | WO 02/085377 A (GELTEX PHARMA INC [US]) 31 October 2002 (2002-10-31) * page 1, line 17 - page 2, line 5 * * page 8, line 27 * * page 31, lines 8-23 * ----- | 1-7 | A61K A61P |
| X | US 2003/175237 A1 (BAILEY WILLIAM L [US] ET AL) 18 September 2003 (2003-09-18) * page 1, right-hand column, paragraphs 2,3 * * page 2, left-hand column, paragraph 2 - right-hand column, paragraph 2 * ----- -/-- | 1-7 | |

The present search report has been drawn up for all claims

1

| Place of search | Date of completion of the search | Examiner |
|---|---|---|
| Munich | 12 May 2011 | Young, Astrid |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 00 2551

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/169146 A1 (GADDAM OM REDDY [IN] ET AL) 14 November 2002 (2002-11-14)<br>* page 1, right-hand column, paragraph 5 - page 2, left-hand column, paragraph 1 *<br>* page 6, left-hand column, paragraph 2 *<br>* claims 48-59 *<br>----- | 1-7 | |
| | | | TECHNICAL FIELDS SEARCHED (IPC) |

The present search report has been drawn up for all claims

1

| Place of search | Date of completion of the search | Examiner |
|---|---|---|
| Munich | 12 May 2011 | Young, Astrid |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 11 00 2551

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2011

| Patent document cited in search report | | Publication date | Patent family member(s) | | | Publication date |
|---|---|---|---|---|---|---|
| WO 03011308 | A1 | 13-02-2003 | AT | 378054 | T | 15-11-2007 |
| | | | CA | 2454732 | A1 | 13-02-2003 |
| | | | CN | 1537010 | A | 13-10-2004 |
| | | | DE | 60223551 | T2 | 23-10-2008 |
| | | | EP | 1413309 | A1 | 28-04-2004 |
| | | | ES | 2295369 | T3 | 16-04-2008 |
| | | | JP | 4353414 | B2 | 28-10-2009 |
| | | | US | 2004191209 | A1 | 30-09-2004 |
| US 5298497 | A | 29-03-1994 | NONE | | | |
| WO 02085377 | A | 31-10-2002 | AT | 390927 | T | 15-04-2008 |
| | | | AU | 2002257145 | B2 | 02-06-2005 |
| | | | BR | 0209134 | A | 15-06-2004 |
| | | | CA | 2444046 | A1 | 31-10-2002 |
| | | | CN | 1511039 | A | 07-07-2004 |
| | | | DE | 60225908 | T2 | 14-05-2009 |
| | | | DK | 1392331 | T3 | 23-06-2008 |
| | | | EP | 1392331 | A1 | 03-03-2004 |
| | | | ES | 2304437 | T3 | 16-10-2008 |
| | | | JP | 4499363 | B2 | 07-07-2010 |
| | | | JP | 2004535384 | T | 25-11-2004 |
| | | | JP | 2010090172 | A | 22-04-2010 |
| | | | MX | PA03009568 | A | 12-02-2004 |
| | | | NZ | 528831 | A | 29-07-2005 |
| US 2003175237 | A1 | 18-09-2003 | NONE | | | |
| US 2002169146 | A1 | 14-11-2002 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03011398 A
- JP 60209523 A
- US 5496545 A
- US 5607669 A
- JP 9504782 A
- JP 9500368 A
- JP 10501264 A
- JP 10501842 A
- JP 11507093 A
- JP 11512074 A
- JP 5512332 A
- JP 8208750 A
- JP 9202732 A
- JP 10114661 A
- JP 11228449 A
- JP 2004091508 A

### Non-patent literature cited in the description

- *Rinsho Iyaku,* June 1996, vol. 12 (8), 1641
- *Ann. Intern. Med.,* 1994, vol. 121, 416-422